# EUROPEAN PATENT APPLICATION

(11) **EP 4 751 724 A1**
(43) Date of publication of application: **03.06.2026**
(21) Application number: 24845224.5
(22) Date of filing: 10.06.2024
(51) Int. Cl.: A61K 35/28, A61K 35/32, A61P 27/04, A61P 37/06

(54) **THERAPEUTIC AGENT OR PROPHYLACTIC AGENT FOR DRY EYE**

(30) Priority: 26.07.2023 JP 2023121228
(71) Applicant: U-Factor Co., Ltd., Tokyo 102-0085 (JP); Keio University, Tokyo, 108-8345 (JP)
(72) Inventor: SHU, Yujing, Tokyo 102-0085 (JP); SETA, Yasuhiro, Tokyo 102-0085 (JP); HORI, Keigo, Tokyo 102-0085 (JP); OTAKE, Masato, Tokyo 102-0085 (JP); SATO, Shinri, Tokyo 160-8582 (JP); SHIMIZU, Eisuke, Tokyo 160-8582 (JP); OGAWA, Yoko, Tokyo 160-8582 (JP); NEGISHI, Kazuno, Tokyo 160-8582 (JP)
(74) Representative: Plasseraud IP
(86) International application number: PCT/JP2024/020970
(87) International publication number: WO 2025/022842

(57) **Abstract**

The present invention aims to provide a novel agent for treating or preventing dry eye, an agent for treating or preventing graft-versus-host disease, an agent for promoting healing of corneal epithelial wound, an agent for suppressing an inflammatory cell infiltrating into the cornea, a tear volume retaining agent, an agent for suppressing a corneal epithelial damage, an agent for suppressing a cell positive for a cell proliferation marker, or an agent for suppressing differentiation of a lymphocyte into an effector T cell. The present invention provides a novel agent for treating or preventing dry eye, an agent for treating or preventing graft-versus-host disease, an agent for promoting healing of corneal epithelial wound, an agent for suppressing an inflammatory cell infiltrating into the cornea, a tear volume retaining agent, an agent for suppressing a corneal epithelial damage, an agent for suppressing a cell positive for a cell proliferation marker, or an agent for suppressing differentiation of a lymphocyte into an effector T cell, the agent comprising a substance derived from a culture supernatant of a stem cell as an active ingredient.

## Description

### [Technical Field]

The present invention relates to an agent for treating or preventing dry eye, an agent for treating or preventing graft-versus-host disease, an agent for suppressing an inflammatory cell infiltrating into the cornea, a tear volume retaining agent, an agent for suppressing a corneal epithelial damage, an agent for suppressing a cell positive for a cell proliferation marker, or an agent for suppressing differentiation of a lymphocyte into an effector T cell.

### [Background Art]

Dry eye is a disease or symptom in which abnormalities in the quantity or composition of tears due to various factors cause dryness or discomfort of the eye, and/or impaired visual function and which becomes chronic with damage to the cornea or conjunctiva on the surface of the eye. It is said that humans obtain more than eighty percent of information through the eyes, and due to dry eye causing chronic symptoms in the eye, their behavior is restricted, their motivation declines, and their quality of life (QOL) is decreased (Non-Patent Document 1).

Dry eye is a disease or symptom with high prevalence. In recent years, due to widespread use of air conditioning, increase in workers using visual display terminals (VDTs), etc., the number of dry eye patients is rapidly increasing; in Japan it is about eight million persons, and worldwide more than ninety million persons, and thus medical needs are increasing. Moreover, it has been reported that the prevalence rate in Asia, including Japan, is higher than in Europe and the United States (Non-Patent Document 2).

Furthermore, the number of symptomatic persons due to aging, wearing contact lenses, and working on computer for a long time, which are risk factors for dry eye, is expected to continue increasing in the future. It is said that continuous view of a monitor screen make tears tend to dry and eye fatigue is induced, leading to decline in visual function called "presbyopia in the evening." There are many causes of dry eye; it is known to also occur as a side effect of anticancer drug therapies. In particular, anti-cancer drugs are thought to affect corneal cells which are highly proliferative, and dry eye is positioned as a high-frequency side effect in many molecular-targeted drugs (Non-Patent Document 2).

It is considered that as the conditions become chronic and severe in dry eye, corneal-conjunctival epithelial damage becomes prominent, leading to various subjective symptoms, due to decrease in tear film stability as one of the core mechanisms. Dry eye is classified into tear-deficient type and evaporative type based on its cause. Meibomian gland dysfunction (MGD) is considered a cause of evaporative dry eye and is the main cause in a considerable proportion of patients presenting to ophthalmology with dry eye symptoms such as ocular discomfort (Non-Patent Document 1). Also, short tear film break-up time (BUT) -type dry eye is characterized by a shortened BUT, normal tear secretion ability, and strong subjective symptoms irrespective of mild ocular surface epithelial damage.

The meibomian gland is a sebaceous gland located within the tarsal plate and having orifices along the upper and lower eyelid margins. Lipids (meibum) secreted from the meibomian glands distribute along the eyelid margins and the outermost layer of tears, functioning to suppress tear evaporation, promote tear stability, facilitate the spreading of tears over the ocular surface, and suppress outflow of tears onto the skin at the eyelid margin, etc. (Non-Patent Document 3).

Meibomian gland dysfunction (MGD) is a term used clinically to refer to a condition in which function of the meibomian glands is impaired (Non-Patent Document 4). However, because (1) there are cases accompanied by inflammation and involvement of resident bacteria and cases not accompanied thereby, and the clinical manifestations are diverse, (2) the severity ranges widely from mild cases to severe cases, (3) there has been no established definition or diagnostic criteria to date, and (4) there are few effective treatments, it has not attracted much attention in general ophthalmic practice. Nevertheless, in fact, in a considerable proportion of patients visiting ophthalmology clinics with ocular discomfort and other symptoms as their chief complaints, MGD is considered to be the cause, leading to a decline in QOL in many patients. Although MGD is a clinically important disease, research on MGD has not progressed much due to the above reasons, and apart from a therapeutic agent for meibomian gland dysfunction comprising active vitamin D3 or its derivative as an active ingredient (Patent Document 1), no effective therapeutic agent has yet been found. At present, treatment of MGD is carried out by thermal therapy or extrusion by applying physical force, in order to improve obstruction of the meibomian glands. In recent years, methods have also been developed in which the lipid layer of the tear film is evaluated by interferometry and the eyelid is treated from the inner side with heat and a massage effect. However, since all these methods must be carried out in medical institutions, a simple treatment method has been awaited.

As described above, dry eye is a chronic ocular disease in which corneal epithelial damage and meibomian gland dysfunction spread to and exacerbate each other, leading to a vicious cycle, with destabilization of the tear film as one of the core mechanisms. Although stratified treatment of the ocular surface, which aims to treat dry eye by supplementing deficient components of the ocular surface to enhance the stability of the tear film, has been proposed, an agent effective only for a part of the layers is insufficient, and a therapeutic agent that is effective in affecting the core mechanisms of dry eye in a multifaceted manner is desired. A therapeutic agent containing apocynin is known as such a therapeutic agent (Patent Document 2), but new therapeutic agents are sought.

Hematopoietic stem cell transplantation is performed as a treatment for curing leukemia by transplanting healthy hematopoietic stem cells from another person in the case of hematologic malignancies caused by abnormalities of hematopoietic stem cells. On the other hand, in recent years, graft-versus-host disease (hereinafter sometimes referred to as GVHD), which develops when a graft of bone marrow cells, peripheral blood, or cord blood provided from a donor attacks the recipient's target organs through an immune response, has become a problem.

To date, it has been reported, using a mouse model exhibiting a pathological condition resembling human chronic GVHD, that mesenchymal stem cells freshly collected from a donor bone marrow possess immunogenicity, engraft in the mucosa of exocrine glands of GVHD, and are involved in the onset of the disease (Non-Patent Document 7). It has also been reported that hematopoietic stem cells transplanted by bone marrow transplantation undergo telomere shortening after transplantation, resulting in stem cell senescence (Non-Patent Document 8). Mesenchymal stem cells were also reported to undergo stem cell senescence in the same way (Non-Patent Document 9).

It is also known that infiltration of senescent macrophages is observed in the lacrimal glands at the early stage of onset in the GVHD mouse model (Non-Patent Document 10). Moreover, it has been shown that the local tissue renin-angiotensin system (RAS) is involved in immune-mediated fibrosis of the GVHD lacrimal glands and other target organs, and that its inhibitors alleviate the pathological condition (Non-Patent Document 11). RAS is considered to induce mitochondrial dysfunction associated with the progression of aging, and it has been suggested that aging-associated changes are involved in the pathology of GVHD. Furthermore, it has been found that endoplasmic reticulum stress associated with aging is excessively enhanced in target organs of chronic GVHD, and it has been reported that administration of the endoplasmic reticulum stress suppressor phenyl butyric acid suppresses inflammation and fibrosis in the target organs (Non-Patent Document 12). These reports suggest that the aging-associated changes are involved in the pathology of GVHD.

After hematopoietic stem cell transplantation, senescent cells accumulate due to excessive stress and decreased immune function by treatment such as radiation exposure (Non-Patent Document 13). In recent years, a mechanism whereby senescent cells continuously release humoral factors and promote activation of adjacent cells to induce chronic inflammation has been reported as the senescence-associated secretory phenotype (SASP) (Non-Patent Documents 14-15). Furthermore, it has been reported that senescent cells are actively involved in the pathogenesis of aging-associated diseases such as macular degeneration (Non-Patent Document 16).

As therapeutic agents for chronic GVHD, immunosuppressive agents are known. The immunosuppressive agents, however, have partial effects and there exist many refractory cases.

As therapeutic agents for GVHD, for example, rebamipide and diquafosol are known for treatment of the ocular surface, and it has been disclosed that dry eye, which is one of the symptoms of GVHD, is improved by combined use of rebamipide and diquafosol. However, although the above-mentioned rebamipide and diquafosol can be used for the treatment of patients with mild and moderate GVHD, they cannot be used for the treatment of patients with severe GVHD. Systemically, treatment with steroids and immunosuppressive agents such as cyclosporine and tacrolimus is performed. However, the effects of treatment with the above-mentioned drugs are still insufficient, and in fact there exist no effective therapeutic agents for GVHD.

It is known that culture supernatants of mesenchymal stem cells (MSCs) contain various growth factors and cytokines, and at present, their usefulness is expected in a wide range of fields from cosmetics to regenerative medicine. MSCs can be readily collected from various tissues such as bone marrow, dental pulp, adipose tissue, and umbilical cord, and in particular, dental pulp stem cells, which are MSCs obtainable from deciduous teeth, can be easily prepared from deciduous teeth that have conventionally been discarded, and furthermore, have high proliferative ability; thus, the dental pulp stem cells are regarded as especially promising MSCs.

To date, it has been reported that culture supernatants of dental pulp stem cells are useful for repair of damaged tissues such as cerebral infarction and for protection of skin tissues (Patent Documents 4-5). In addition, as an attempt to treat dry eye using cell culture supernatants, Patent Document 6 describes a pharmaceutical composition for use in the treatment of dry eye disease, comprising a secretome of amniotic fluid stem cells prepared by a method including: culturing amniotic fluid stem cells in basal medium for 24-72 hours to obtain a culture medium; collecting the culture supernatant after centrifugation; and filtering the supernatant to obtain the secretome.

Further, Patent Documents 7-8 describe a regenerative therapeutic composition comprising a culture supernatant of a dental pulp-derived stem cell, which does not contain the dental pulp-derived stem cell itself, characterized in that the concentration of vascular endothelial growth factor (VEGF), which is a cytokine contained in the cell culture supernatant of the dental pulp-derived stem, is 150-5000 pg/ml, and also describe the use of the regenerative therapeutic composition for improvement or prevention of dry eye. However, even if a person skilled in the art can grasp an invention of a product from matters described in a publication and matters equivalent to those described, the invention cannot be regarded as a cited invention in the case that it is not clear, on the basis of the description of the publication and the common general knowledge at the time of filing, that a person skilled in the art can make the product. In Patent Documents 7-8, no examples showing that the regenerative therapeutic composition is used for improvement or prevention of dry eye are described and the regenerative therapeutic composition is not described in such a manner that it is clear that the regenerative therapeutic composition for improvement or prevention of dry eye can be produced. Therefore, Patent Documents 7-8 lacked eligibility for recognizing inventions of a culture supernatant of a dental pulp-derived stem cell for improvement or prevention of dry eye as cited inventions.

Furthermore, in Patent Document 9, a sex hormone secretion promoter that promotes a sex hormone secretion is described, the promoter comprising a culture supernatant of an umbilical cord-derived stem cell obtained by culturing the umbilical cord-derived stem cell, and the sex hormone secretion promoter is described to be used for improvement or prevention of dry eye. However, in Patent Document 9, like Patent Documents 7-8, no examples showing that the sex hormone secretion promoter is used for improvement or prevention of dry eye are described, and the sex hormone secretion promoter is not described in such a manner that it is clear that the sex hormone secretion promoter for improvement or prevention of dry eye can be produced. Therefore, Patent Document 9 lacked eligibility for recognizing inventions of a culture supernatant of a umbilical cord-derived stem cells for improvement or prevention of dry eye as cited inventions.

### [References]

### [Patent Documents]

[Patent Document 1] International Publication No. 2014/208709
[Patent Document 2] International Publication No. 2022/065436
[Patent Document 3] Japanese Patent Application Publication No. 2020-111548
[Patent Document 4] International Publication No. 2011/118795
[Patent Document 5] International Publication No. 2014/126176
[Patent Document 6] Japanese Patent Application Publication No. 2022-184814
[Patent Document 7] Japanese Patent Application Publication No. 2021-038268
[Patent Document 8] International Publication No. 2020/130038
[Patent Document 9] Japanese Patent Application Publication No. 2017-119646

### [Non-Patent Documents]

[Non-Patent Document 1] Kojima et al., Progress in Retinal and Eye Research, p. 100842, 2020; Borkar et al., Support Care Cancer 21:1167-1174 (2013)
[Non-Patent Document 2] Yang et al., Int J Mol Med 41:1427-1436 (2018); Lin et al., Mol Vis 17:257-264 (2011)
[Non-Patent Document 3] Foulks G.N., Bron A.J., Ocul Surf 1:107-126, 2003.
[Non-Patent Document 4] Gutgesell V.J., Stern G.A., Hood C.I., Am J Ophthalmol 94:383-387, 1982.
[Non-Patent Document 5] Chang J., et al., Nat Med 2016 Jan;22(1):78-83. doi:10.1038/nm.4010. Epub 2015 Dec 14.
[Non-Patent Document 6] FASEB J. 2020 Aug;34(8):10778-10800. doi:10.1096/fj.201900218R. Epub 2020 Jul 3.
[Non-Patent Document 7] Ogawa Y., et al., eLife, 2016 Jan 26;5:e09394. doi:10.7554/eLife.09394.
[Non-Patent Document 8] Seligman S.J., Lancet, 1998 Apr 25;351(9111):1287-8.
[Non-Patent Document 9] Sethe S., Aging Res Rev, 2006 Feb;5(1):91-116. Epub 2005 Nov 28.
[Non-Patent Document 10] Kawai M., Ogawa Y., et al., Sci Rep, 2013;3:2455. doi:10.1038/srep02455.
[Non-Patent Document 11] Yaguchi S., Ogawa Y., et al., PLoS One, 2013 Jun 6;8(6):e64724. doi:10.1371/journal.pone.0064724. Print 2013.
[Non-Patent Document 12] Mukai S., Ogawa Y., et al., Sci Rep 2017; Royal Society Open Science, 2018 Oct 17;5(10):181067. doi:10.1098/rsos.181067. eCollection 2018 Oct.
[Non-Patent Document 13] Munoz-Espin D., Nat Rev Mol Cell Biol, 2014 Jul;15(7):482-96. doi:10.1038/nrm3823.
[Non-Patent Document 14] Rodier F., J Cell Biol. 2011 Feb 21;192(4):547-56. doi:10.1083/jcb.201009094. Epub 2011 Feb 14.
[Non-Patent Document 15] Ohtani N., Nature 2013 Jul 4;499(7456):97-101. doi:10.1038/nature12347. Epub 2013 Jun 26.
[Non-Patent Document 16] Apte R.S., Cell Metab. 2013 Apr 2;17(4):549-61. doi: 10.1016/j.cmet.2013.03.009.

### [Summary of the Invention]

### [Problem to be Solved by the Invention]

Accordingly, an object of the present invention is to provide a new agent for treating or preventing dry eye, a new agent for treating or preventing graft-versus-host disease, a new agent for promoting healing of corneal epithelial wound, a new agent for suppressing an inflammatory cell infiltrating into the cornea, a new tear volume retaining agent, a new agent for suppressing a corneal epithelial damage, a new agent for suppressing a cell positive for a cell proliferation marker, or a new agent for suppressing differentiation of a lymphocyte.

### [Means for Solving the Problem]

The inventors of the present invention, as a result of intensive studies, have found that a substance derived from a culture supernatant of a stem cell can serve as an active ingredient of an agent for treating or preventing dry eye, an agent for treating or preventing graft-versus-host disease, an agent for promoting healing of corneal epithelial wound, an agent for suppressing an inflammatory cell infiltrating into the cornea, a tear volume retaining agent, an agent for suppressing a corneal epithelial damage, an agent for suppressing a cell positive for a cell proliferation marker, or an agent for suppressing differentiation of a lymphocyte into an effector T cell.

That is, the present invention provides the followings.

### [Aspect 1]

An agent for treating or preventing dry eye, an agent for treating or preventing graft-versus-host disease, an agent for promoting healing of corneal epithelial wound, an agent for suppressing an inflammatory cell infiltrating into the cornea, a tear volume retaining agent, an agent for suppressing a corneal epithelial damage, an agent for suppressing a cell positive for a cell proliferation marker, or an agent for suppressing differentiation of a lymphocyte into an effector T cell, the agent comprising a substance derived from a culture supernatant of a stem cell as an active ingredient.

### [Aspect 2]

The agent of Aspect 1, wherein the graft-versus-host disease is graft-versus-host disease in the eye.

### [Aspect 3]

The agent of Aspect 1, wherein the stem cell is a dental pulp stem cell.

### [Aspect 4]

The agent of Aspect 3, wherein the dental pulp stem cell is a deciduous tooth-derived dental pulp stem cell.

### [Aspect 5]

The agent of Aspect 1, wherein the stem cell is a primary cell.

### [Aspect 6]

The agent of Aspect 1, wherein the stem cell is an immortalized cell.

### [Aspect 7]

The agent of Aspect 1, wherein the substance derived from the culture supernatant of the stem cell is a substance obtained by a method comprising the step of removing components of molecular weight less than 3.5 kDa from the culture supernatant of the stem cell.

### [Aspect 8]

The agent of Aspect 7, wherein the method comprising the step of removing components of molecular weight less than 3.5 kDa from the culture supernatant of the stem cell is a method of using a dialysis membrane.

### [Aspect 9]

The agent of Aspect 1, wherein the substance derived from the culture supernatant of the stem cell does not comprise components of molecular weight less than 3.5 kDa.

### [Aspect 10]

The agent of Aspect 1, wherein the protein concentration of the substance derived from the culture supernatant of the stem cell is 30 µg/mL to 100 µg/mL.

### [Aspect 11]

The agent of Aspect 1, wherein the substance derived from the culture supernatant of the stem cell comprises an extracellular vesicle.

### [Aspect 12]

The agent of Aspect 1, wherein the substance derived from the culture supernatant of the stem cell comprises an exosome.

### [Effects of the Invention]

According to the present invention, differentiation of lymphocytes into effector T cells can be suppressed, cells positive for cell proliferation markers can be suppressed, corneal epithelial damage can be suppressed, tear volume can be retained, inflammatory cells infiltrating into the cornea can be suppressed, healing of corneal epithelial wounds can be promoted, graft-versus-host disease can be treated or prevented, and dry eye can be treated or prevented.

### [Brief Description of the Drawings]

[Fig. 1] Fig. 1 shows the measurement results of corneal epithelial damage scores in the control mouse group, the 3.5 kD Hi eye drop group, the 3.5 kD Lo eye drop group, and the control solution administration group, at 28th Day after radiation exposure.
[Fig. 2] Fig. 2 shows the measurement results of tear volume in the control mouse group, the 3.5 kD Hi eye drop group, the 3.5 kD Lo eye drop group, and the control solution administration group at 28th Day after radiation exposure.
[Fig. 3] Fig. 3 shows the measurement results of the number of inflammatory cells infiltrating into the cornea in the control mouse group, the 3.5 kD Hi eye drop group, the 3.5 kD Lo eye drop group, and the control solution administration group at 28th day after radiation exposure.
[Fig. 4] Fig. 4 shows the analysis results of corneal epithelial defect area in the 3.5 kD Hi eye drop group, the 3.5 kD Lo eye drop group, and the control solution administration group at 72 hours after corneal epithelial scraping in mice.
[Fig. 5] Fig. 5 shows the results of lymphocyte activation experiments using spleen-derived immune cells. Fig. 5(a) shows the suppressive effect of the present agent on cell proliferation marker-positive CD4 T cells. Fig. 5(b) shows the suppressive effect of the present agent on differentiation of lymphocytes into IFNγ-positive effector Th1 cells.

### [Mode for Carrying Out the Invention]

The present invention provides an agent for treating or preventing dry eye, an agent for treating or preventing graft-versus-host disease, an agent for promoting healing of corneal epithelial wound, an agent for suppressing an inflammatory cell infiltrating into the cornea, a tear volume retaining agent, an agent for suppressing a corneal epithelial damage, an agent for suppressing a cell positive for a cell proliferation marker, or an agent for suppressing differentiation of a lymphocyte into an effector T cell, the agent comprising a substance derived from a culture supernatant of a stem cell as an active ingredient.

In the expression "a substance derived from a culture supernatant of a stem cell", the stem cell is preferably a dental pulp stem cells and the dental pulp stem cell is preferably a deciduous tooth dental pulp stem cell. The deciduous tooth dental pulp stem cell is, for example, a mesenchymal stem cell collected from the dental pulp of deciduous teeth. Methods for preparing deciduous tooth dental pulp stem cells have been already known (for example, see International Publication No. 2011/118795), and for example, may be isolated and prepared from deciduous tooth dental pulp in accordance with publicly known methods in the art.

The stem cell is preferably a mesenchymal stem cell. The stem cell may be a primary cell or may be an immortalized cell. The immortalized cell is, for example, a cell that maintains a state capable of proliferating even after a certain number of divisions, for example, a cell having infinite proliferative capacity. Methods for preparing immortalized cells have already been established and, for example, known techniques may be employed. For example, immortalized cells can be prepared by introducing into cells genes such as an SV40T antigen gene or a telomerase reverse transcriptase (TERT) gene.

The stem cell may be a stem cell derived from any vertebrate, and preferably a stem cells derived from mammals such as mouse, rat, rabbit, pig, cattle, monkey, or human, and more preferably a stem cell derived from human.

In a method for preparing the culture supernatant of the stem cell, culture of the stem cell can be carried out, for example, in accordance with publicly known methods in the art (for example, see International Publication No. 2011/118795). Specifically, for example, stem cells may be cultured by seeding them at a density of, for example, 5×10⁴ to 2×10⁵ cells/mL in a medium obtained by appropriately supplementing serum such as FBS or a serum substitute such as KSR, glucose, amino acids, vitamins, and the like to a basal medium such as DMEM, IMDM, Ham's F-12, or RPMI-1640 or a mixture thereof. The culture of stem cells is preferably carried out for 0 to 15 passages in the case that the stem cells are primary cultured cells, and preferably for 30 to 100 passages in the case that the stem cells are immortalized cells. The culture supernatant of stem cells is preferably a culture supernatant obtained by culturing stem cells in a serum-free medium. The culture of stem cells in serum-free medium is carried out, for example, for 24 to 72 hours. The culture supernatant of stem cells can be obtained, for example, by collecting the medium in which the stem cells have been cultured and filtering it using a separation membrane. The separation membrane is preferably 0.1 µm to 1 µm, more preferably 0.1 µm to 0.5 µm, and still more preferably 0.1 µm to 0.3 µm.

The substance derived from the culture supernatant of the stem cell may be the culture supernatant of the stem cell itself or may be a concentrate of the culture supernatant of the stem cell. In the case that the substance derived from culture supernatant of the stem cell is a concentrate of the culture supernatant of the stem cell, the substance derived from the culture supernatant of the stem cell is preferably a 2-fold to 100-fold concentrate of the culture supernatant of the stem cell, more preferably a 3-fold to 50-fold concentrate of the culture supernatant of the stem cell, and still more preferably a 5-fold to 20-fold concentrate of the culture supernatant of the stem cell. Here, concentration of the culture supernatant of the stem cell is performed, for example, by ultrafiltration. The substance derived from the culture supernatant of the stem cell is preferably a substance obtained by a method comprising a step of removing components having a molecular weight of less than 3.5 kDa from the culture supernatant of the stem cell. Here, the kDa may be calculated, for example, on the basis of SDS-PAGE or ultrafiltration, and in the case that the culture supernatant is purified using a commercially available ultrafiltration filter unit, it may be the nominal molecular weight limit (NMWL) or molecular weight cut-off (MWCO) value indicated on the product. A method comprising a step of removing components having a molecular weight of less than 3.5 kDa from the culture supernatant of the stem cell is, for example, a method using a dialysis membrane. Examples of dialysis membranes include, for example, dialysis membranes with 1,000 MWCO to 10,000 MWCO, preferably a dialysis membrane with 3,500 MWCO. In the case that the substance derived from the culture supernatant of the stem cell is a substance obtained by a method comprising a step of removing components having a molecular weight of less than 3.5 kDa from the culture supernatant of the stem cell, the substance derived from culture supernatant of the stem cell may further be a concentrate of a substance obtained by a method comprising a step of removing components having a molecular weight of less than 3.5 kDa from the culture supernatant of the stem cell. Here, the concentration is preferably a 2-fold to 100-fold concentration, more preferably a 3-fold to 50-fold concentration, and still more preferably a 5-fold to 20-fold concentration. The concentration is carried out, for example, by ultrafiltration.

In the case that the substance derived from the culture supernatant of the stem cell is a substance obtained by a method comprising a step of removing components having a molecular weight of less than 3.5 kDa from the culture supernatant of the stem cell, the substance derived from the culture supernatant of the stem cell may be any substance obtained by a method comprising a step for the purpose of removing components having a molecular weight of less than 3.5 kDa from the culture supernatant of a stem cell, and therefore, after undergoing the method, it is not negated, by the mere fact that, for example, due to the technical limitations of the method, components having a molecular weight of less than 3.5 kDa remain without being completely removed, that the substance derived from the culture supernatant of the stem cell is a substance obtained by a method comprising a step of removing components having a molecular weight of less than 3.5 kDa from the culture supernatant of the stem cell. Moreover, in the case that the substance derived from the culture supernatant of the stem cell is a substance obtained by a method comprising a step of removing components having a molecular weight of less than 3.5 kDa from the culture supernatant of a stem cell, the substance derived from the culture supernatant of the stem cell may be a substance in which components having a molecular weight of less than 3.5 kDa have been appropriately added to the substance obtained by the method, and therefore, by the mere detection of components having a molecular weight of less than 3.5 kDa it is not negated that the substance derived from culture supernatant of a stem cell is a substance obtained by a method comprising a step of removing components having a molecular weight of less than 3.5 kDa from the culture supernatant of the stem cell. However, the substance derived from culture supernatant of the stem cell preferably substantially does not contain components having a molecular weight of less than 3.5 kDa, and more preferably contains no components having a molecular weight of less than 3.5 kDa.

The protein concentration in the substance derived from the culture supernatant of the stem cell is preferably 5 µg/mL to 1,000 µg/mL, more preferably 5 µg/mL to 500 µg/mL, still more preferably 5 µg/mL to 300 µg/mL, still more preferably 5 µg/mL to 200 µg/mL, and still more preferably 5 µg/mL to 100 µg/mL. The protein concentration in the substance derived from the culture supernatant of the stem cell is also preferably 10 µg/mL to 1,000 µg/mL, more preferably 10 µg/mL to 500 µg/mL, still more preferably 10 µg/mL to 300 µg/mL, still more preferably 10 µg/mL to 200 µg/mL, and still more preferably 10 µg/mL to 100 µg/mL. The protein concentration in the substance derived from the culture supernatant of the stem cell is also preferably 15 µg/mL to 1,000 µg/mL, more preferably 15 µg/mL to 500 µg/mL, still more preferably 15 µg/mL to 300 µg/mL, still more preferably 15 µg/mL to 200 µg/mL, and still more preferably 15 µg/mL to 100 µg/mL. The protein concentration in the substance derived from the culture supernatant of the stem cell is also preferably 20 µg/mL to 1,000 µg/mL, more preferably 20 µg/mL to 500 µg/mL, still more preferably 20 µg/mL to 300 µg/mL, still more preferably 20 µg/mL to 200 µg/mL, and still more preferably 20 µg/mL to 100 µg/mL. The protein concentration in the substance derived from the culture supernatant of the stem cell is also preferably 30 µg/mL to 1,000 µg/mL, more preferably 30 µg/mL to 500 µg/mL, still more preferably 30 µg/mL to 300 µg/mL, still more preferably 30 µg/mL to 200 µg/mL, and still more preferably 30 µg/mL to 100 µg/mL. Here, the protein concentration in the substance derived from the culture supernatant of the stem cell is measured by a protein assay kit.

The substance derived from the culture supernatant of the stem cell preferably contains extracellular vesicles (EVs). Examples of extracellular vesicles include, for example, exosomes, microvesicles, and apoptotic bodies. The molecular weight of extracellular vesicles (EVs) is 100 kDa or more, and the size of extracellular vesicles is approximately 50 nm to 200 nm. EVs are complexes of molecules, and their components include not only various proteins but also nucleic acids such as microRNA and mRNA. Therefore, even in the case that the substance derived from the culture supernatant of the stem cell is a substance obtained by a method comprising a step of removing components having a molecular weight of less than 3.5 kDa from the culture supernatant of the stem cell, it contains extracellular vesicles (EVs), such as exosomes.

As shown in the Examples described later, administration of the substance derived from the culture supernatant of the stem cell to a subject can suppress differentiation of lymphocytes into effector T cells in the subject, can suppress cells positive for cell proliferation markers in the subject, can suppress corneal epithelial damage in the subject, can retain tear volume in the subject, can suppress inflammatory cells infiltrating into the cornea in the subject, can promote healing of corneal epithelial wounds in the subject, can treat or prevent graft-versus-host disease in the subject, and can treat or prevent dry eye in the subject. Examples of suppressing differentiation of lymphocytes into effector T cells in the subject include suppressing differentiation of lymphocytes into effector helper T cells in the subject. Examples of suppressing differentiation of lymphocytes into effector helper T cells in the subject include suppressing differentiation of lymphocytes into IFNγ-positive effector Th1 cells in the subject. It should be noted that IFNγ is a cytokine that induces inflammation. Examples of suppressing cells positive for cell proliferation markers in the subject include suppressing CD4-positive T cells positive for cell proliferation markers in the subject. Examples of suppressing cells positive for cell proliferation markers in the subject also include suppressing lymphocytes positive for cell proliferation markers in the subject. The graft-versus-host disease is, for example, graft-versus-host disease in the eye.

The subject may be any vertebrate, for example, but is preferably a mammal such as mouse, rat, rabbit, pig, cattle, monkey, or human, and more preferably is human. The subject may be of any age, including infants, children, adolescents, adults, and the elderly.

An agent for treating or preventing dry eye, an agent for treating or preventing graft-versus-host disease, an agent for promoting healing of corneal epithelial wound, an agent for suppressing an inflammatory cell infiltrating into the cornea, a tear volume retaining agent, an agent for suppressing a corneal epithelial damage, an agent for suppressing a cell positive for a cell proliferation marker, or an agent for suppressing differentiation of a lymphocyte into an effector T cell (hereinafter referred to as "the agent of the present invention") may consist of the culture supernatant of the stem cell itself or a fraction purified from the culture supernatant of the stem cell, but may further comprise, as optional components, pharmaceutically acceptable carriers and additives.

The agent of the present invention may be prepared as a liquid agent or a solid agent, and may be formulated into various dosage forms such as injections, jellies, sprays, tablets, or granules, but is preferably prepared as a liquid preparation. In preparing a liquid agent, carriers such as sterile water, physiological saline, or glucose solution can be used, and bactericides, isotonic agents, stabilizers, and the like may further be added as further desired. In preparing a solid agent, additives such as starch, lactose, mannitol, and inorganic salts, and, if further desired, binders, disintegrants, lubricants, and the like may be added.

The content (in terms of protein weight) of the substance derived from the culture supernatant of the stem cell in the agent of the present invention, when the total weight of the agent of the present invention is 100%, may be, for example, 60% by weight or more, 70% by weight or more, 80% by weight or more, or 90% by weight or more, and preferably 60% to 70% by weight.

The agent of the present invention may be administered by any administration method suitable for the dosage form, for example, oral administration, intravenous administration, subcutaneous administration, transdermal administration, intramuscular administration, intranasal administration, intraperitoneal administration, direct injection into target tissues, or inhalation administration. The dosage of the agent of the present invention may vary depending on the age, body weight, health condition, and the like of the subject, but may be, for example, 0.1 to 10 mg/kg (body weight), preferably 0.5 to 2 mg/kg (body weight). The dosage may be administered in a single dose or in multiple doses.

The present invention provides a method for treating or preventing dry eye in a subject, a method for treating or preventing graft-versus-host disease in a subject, a method for promoting corneal epithelial wound healing in a subject, a method for suppressing an inflammatory cell infiltrating into the cornea in a subject, a method for retaining tear volume in a subject, a method for suppressing corneal epithelial damage in a subject, a method for suppressing a cell positive for a cell proliferation marker in a subject, or a method for suppressing differentiation of a lymphocyte into an effector T cell in a subject, characterized by administering to the subject a substance derived from a culture supernatant of a stem cell. The present invention also provides a pharmaceutical composition for use as An agent for treating or preventing dry eye, an agent for treating or preventing graft-versus-host disease, an agent for promoting healing of corneal epithelial wound, an agent for suppressing an inflammatory cell infiltrating into the cornea, a tear volume retaining agent, an agent for suppressing a corneal epithelial damage, an agent for suppressing a cell positive for a cell proliferation marker, or an agent for suppressing differentiation of a lymphocyte into an effector T cell, comprising a substance derived from a culture supernatant of a stem cell as an active ingredient. Furthermore, the present invention provides a use of a substance derived from a culture supernatant of a stem cell in the manufacture of a pharmaceutical composition for use as an agent for treating or preventing dry eye, an agent for treating or preventing graft-versus-host disease, an agent for promoting healing of corneal epithelial wound, an agent for suppressing an inflammatory cell infiltrating into the cornea, a tear volume retaining agent, an agent for suppressing a corneal epithelial damage, an agent for suppressing a cell positive for a cell proliferation marker, or an agent for suppressing differentiation of a lymphocyte into an effector T cell.

### [Examples]

### <Materials and Reagents>

NIH3T3 cells were purchased from JCRB Cell Bank. Human umbilical vein endothelial cells (HUVEC), EBM^{™}-2 basal medium (CC-3156), and EGM^{™}-2 supplement kit (CC-4176) were purchased from Lonza. Dulbecco's Modified Eagle Medium (DMEM) was purchased from GIBCO. Bovine calf serum (hereinafter referred to as "CS") and antifungal solution (100×) were purchased from Sigma-Aldrich. Fetal bovine serum (FBS) and 0.05% trypsin/EDTA solution were purchased from Invitrogen. Charcoal (powder, activated) (hereinafter referred to as "activated charcoal") was purchased from Nacalai Tesque. Dulbecco's phosphate-buffered saline (PBS), RIPA buffer, and Quick CBB staining solution were purchased from FUJIFILM Wako Pure Chemical Corporation. Ultrafiltration units were purchased from SARTORIUS. Millex-GV low protein binding Durapore (PVDF) Membrane 0.22 µm was purchased from Merck Millipore. Spectra/Por^{™} 7 Dialysis Membrane (MWCO: 3.5 kD) was purchased from REPLIGEN. PD-10 columns were purchased from Cytiva. Micro BCA Protein Assay Kit was purchased from Thermo Fisher Scientific. Cell Counting Kit-8 was purchased from Dojindo Laboratories. Acrylamide, protein standard marker, and Clarity Western ECL Substrate were purchased from Bio-Rad. Protease inhibitor complete (#11 697 498 001) was purchased from Roche. Anti-phospho p44/42 antibody (#9106) and anti-phospho Akt antibody (#9271) were purchased from Cell Signaling Technology. Anti-mouse IgG-HRP antibody (#62-6520) and anti-rabbit IgG-HRP antibody (#65-6120) were purchased from Invitrogen.

### [Example 1]

Preparation of culture supernatant of dental pulp stem cells derived from deciduous teeth:

### (1) Preparation of human dental pulp stem cells derived from deciduous teeth (SHED)

After extracted or exfoliated human deciduous teeth were disinfected with 5% chlorhexidine solution or 10% povidone-iodine solution, their crowns were sectioned, and dental pulp tissues were collected using a dental reamer. The pulp tissues were suspended in DMEM containing 10 vol% FBS, to which 2 mg/mL of collagenase and dispase were added, and then incubated at 37°C for 1 hour. Centrifugation was performed at 777 × g for 5 minutes, the supernatant was removed, and pulp cells were collected. The pulp cells were suspended in 4 mL of DMEM supplemented with 10 vol% FBS and antifungal agent and seeded onto a 6-well plate. The cells were incubated at 37°C under 5% CO₂ until sub-confluent. A 0.05% trypsin/EDTA solution was added to the cells, which were then incubated at 37°C for 5 minutes. The cells were collected and seeded onto 10 cm culture dishes for adherent cells (Violoamo). Thereafter, the cells were subcultured three times and proliferated to approximately 1 × 10⁷ cells/mL. The cells collected by addition of 0.05% trypsin/EDTA solution and incubation at 37°C for 5 minutes were designated as "SHED (Stem cells from Human Exfoliated Deciduous teeth)."

### (2) Preparation of immortalized human dental pulp stem cells derived from deciduous teeth (IM-SHED)

SHED immortalized by introduction of the SV40 gene from a viral vector (IM-SHED) was prepared.

### (3) Preparation of culture supernatant

SHED or IM-SHED prepared as described above was subcultured in DMEM containing 10 vol% FBS. For preparation of the culture supernatant, SHED within 8 passages and IM-SHED between 50 and 55 passages were used. The medium was replaced with serum-free DMEM, and the cells were cultured for 48 hours. Thereafter, the medium was collected and filtered with a separation membrane (Stericup Quick Release-GP, PVDF, 0.22 µm; Merck Millipore) to obtain culture supernatant (conditioned medium: CM). Hereinafter, the culture supernatant derived from SHED is referred to as "SHED-CM," and the culture supernatant derived from IM-SHED is referred to as "IM-SHED-CM."

### [Example 2]

### Preparation of test solutions:

IM-SHED-CM (protein concentration: 107 µg/mL) was enclosed in a Spectra/Por^{™} 7 dialysis membrane (MWCO: 3.5 kDa) and dialyzed against 100-fold volume of Milli-Q water for 72 hours. During this period, the Milli-Q water was replaced 8 times. The solution after dialysis was lyophilized, dissolved in 1/10 of the starting volume of PBS (on ice, >30 minutes), and filtered through a 0.22 µm PVDF filter. Thus, a ≥3.5 kDa fraction was obtained. In this manner, a fractioned extract was prepared using the immortalized dental pulp culture supernatant of the stem cell derived from deciduous teeth (IM-SHED-CM), from which components of less than 3.5 kDa molecular weight had been removed. To the model animals described below, eye drops of a 10-fold concentrate of the fraction extract from which components of less than 3.5 kDa molecular weight had been removed (protein concentration: 427 µg/mL) (3.5 kD Hi) and an undiluted solution equivalent concentration (protein concentration: 42.7 µg/mL) (3.5 kD Lo) were administered. The protein concentration was measured using a protein assay kit. Dilution of the extract concentration was performed with PBS. PBS was also used as a control solution for eye drop administration.

### Production of chronic graft-versus-host disease model animals:

Major Histocompatibility Complex (MHC)-compatible but minor histocompatibility antigen (miHA)-incompatible spleen cells and bone marrow cells from male mice (B10.D2, 7-9 weeks old; Sankyo Laboratory) were transplanted to Female mice (BALB/c, 7-9 weeks old; Sankyo Laboratory). As control mice, female mice (BALB/c) to which syngeneic spleen cells and bone marrow cells had been transplanted were used. As the transplantation method, after irradiating the recipient with 7 Gy of radiation, 1 × 10⁶ bone marrow cells and 2 × 10⁶ spleen cells were suspended in 0.1 mL of Roswell Park Memorial Institute medium (RPMI medium), and a total of 0.2 mL was administered into tail vein. The model mice were housed for 7 days with sterilized water and feed under SPF (specific pathogen free) conditions, and then administration of the test agent was initiated. One drop (approximately 5 µL) of the above-described 3.5 kD Hi and 3.5 kD Lo eye drop solutions was administered twice a day for 21 consecutive days. For comparison, control solution (PBS) was similarly administered. At 28 days after irradiation, the corneal epithelium was observed by fluorescein staining of the cornea and the mice were sacrificed and tissues were collected.

### Observation of healing of the wound by corneal epithelial scraping in mice:

The corneal epithelium of female mice (BALB/c, 12 weeks old; Sankyo Laboratory) was scraped in a 3 mm diameter area and one drop (approximately 5 µL) of the above-described 3.5 kD Lo eye drop solution was administered three times a day for 3 consecutive days. For comparison, control solution (PBS) was similarly administered. The corneal epithelium was observed by fluorescein staining of the cornea and the process of healing of the corneal epithelial wound was observed.

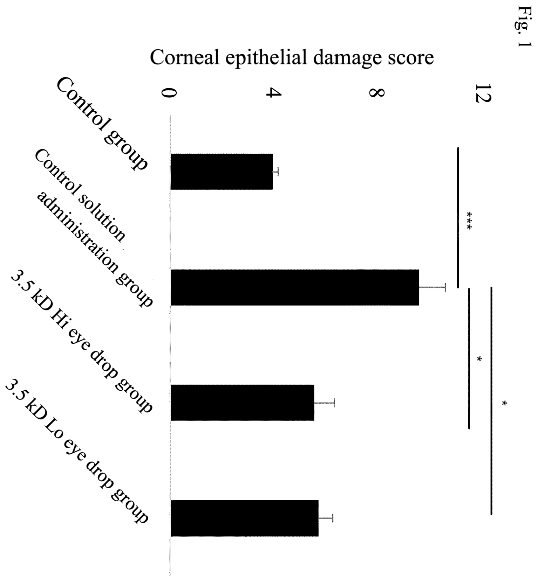

Fig. 1 shows the measurement results of corneal epithelial damage scores in the control mouse group, the 3.5 kD Hi eye drop group, the 3.5 kD Lo eye drop group, and the control solution administration group at 28 days after irradiation. The ocular surface was stained with fluorescein, the staining images were scored in four grades, and the sum of scores of four regions of the cornea (nasal-dorsal, nasal-ventral, temporal-dorsal, temporal-ventral) was calculated, and the mean ± standard error was taken as the corneal epithelial damage score. The results were 5.5 ± 0.77 points in the 3.5 kD Hi group, 5.7 ± 0.54 points in the 3.5 kD Lo group, and 9.5 ± 1.5 points in the control solution eye drop group; and both the 3.5 kD Hi group and the 3.5 kD Lo group showed significantly better corneal epithelial damage scores compared to the control solution eye drop group (p = 0.015, 0.032, n = 3-5 per group, one-way analysis of variance (ANOVA) with Tukey-Kramer's post hoc test, *P < 0.05, **P < 0.01, ***P < 0.001). The scoring criteria were as follows:
0: no staining
1: fewer than 30 punctate corneal epithelial lesions
2: diffuse punctate corneal epithelial lesions observed
3: formation of corneal plaques observed

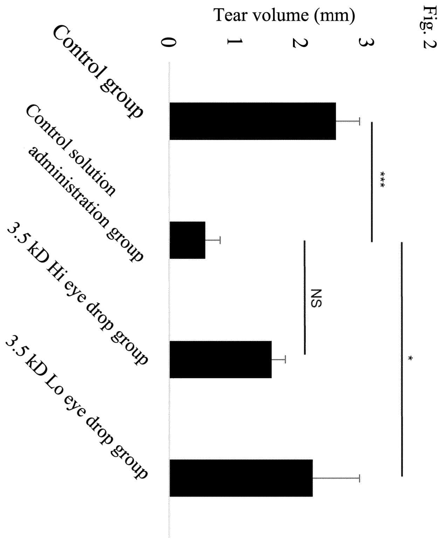

Fig. 2 shows the measurement results of tear volume in the control mouse, 3.5 kD Hi eye drop group, the 3.5 kD Lo eye drop group, and the control solution administration group at 28 days after irradiation. Phenol red thread was inserted into the lateral canthus for 15 seconds, and the length of the stained portion was measured, and the mean ± standard error was taken as the tear volume of each group. The results were 1.56 ± 0.20 in the 3.5 kD Hi group, 2.19 ± 0.73 in the 3.5 kD Lo group, and 0.55 ± 0.23 in the control solution eye drop group; and the 3.5 kD Hi group showed a favorable trend compared with the control solution eye drop group (p = 0.82, n = 3-5 per group, one-way ANOVA with Tukey-Kramer's post hoc test) and the 3.5 kD Lo group showed significantly better corneal epithelial damage scores compared with the control solution eye drop group (p = 0.023, n = 3-5 per group, one-way ANOVA with Tukey-Kramer's post hoc test, *P < 0.05, **P < 0.01, ***P < 0.001).

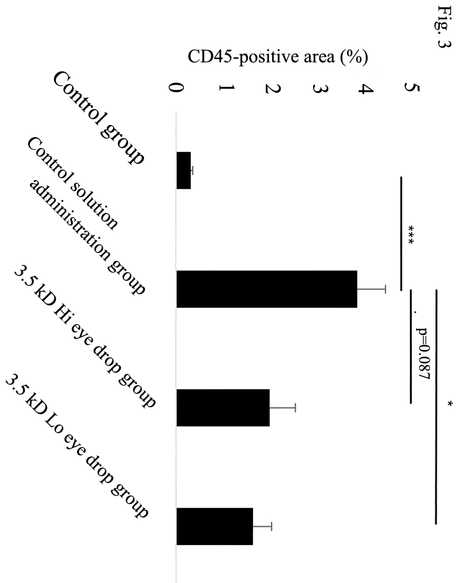

Fig. 3 shows the measurement results of the number of inflammatory cells infiltrating into the cornea in the control mouse group, the 3.5 kD Hi eye drop group, the 3.5 kD Lo eye drop group, and control solution administration group at 28 days after irradiation. Using the entire cornea, inflammatory cell marker CD45 was immunohistochemically labeled, and the CD45-positive area relative to the entire cornea was analyzed using the image analysis software ImageJ. The mean ± standard error of each group was taken as the CD45-positive area. The results were 2.0 ± 0.54 (%) in the 3.5 kD Hi group, 1.63 ± 0.41 (%) in the 3.5 kD Lo group, and 3.9 ± 0.60 (%) in the control solution eye drop group; and the 3.5 kD Hi group showed a favorable trend compared with the control solution eye drop group (p = 0.087, n = 3-5 per group, one-way ANOVA with Tukey-Kramer's post hoc test), and the 3.5 kD Lo group showed significantly better corneal epithelial damage scores compared with the control solution eye drop group (p = 0.046, n = 3-5 per group, one-way ANOVA with Tukey-Kramer's post hoc test, *P < 0.05, **P < 0.01, ***P < 0.001).

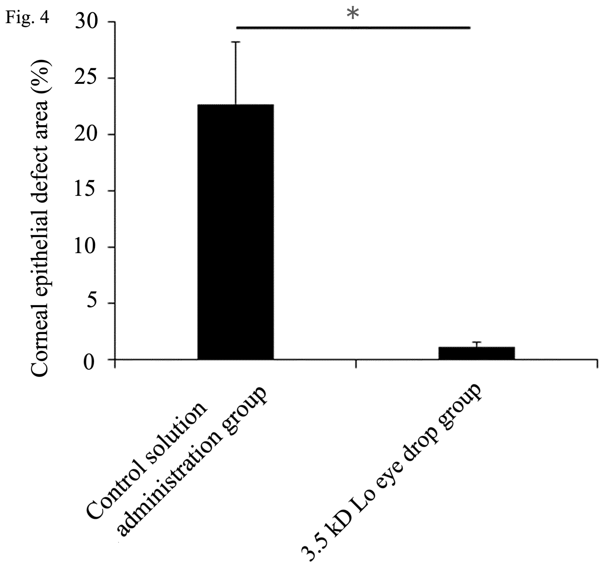

Fig. 4 shows the analysis results of corneal epithelial defect area at 72 hours after corneal epithelial scraping in mice in the 3.5 kD Lo eye drop group and the control solution administration group. The ocular surface was stained with fluorescein, and the percentage of corneal epithelial defect area relative to the entire cornea was analyzed using the image analysis software ImageJ. The mean ± standard error of each group was taken as the corneal epithelial defect area. The results were 1.21% ± 0.39% in the 3.5 kD Lo group and 22.7 ± 5.5% in the control solution administration group, showing that the corneal epithelial defect area was significantly reduced in the 3.5 kD Lo group (p = 0.015, n = 5 per group, unpaired t-test, *P < 0.05, **P < 0.01, ***P < 0.001).

In summary, IM-SHED-CM extract exhibited corneal epithelial damage suppressive effect, tear volume retention effect, suppressive effect on inflammatory cell infiltrating into the cornea, and corneal epithelial wound healing promoting effect in chronic GVHD model mice, suggesting that it has suppressive effects on ocular GVHD and dry eye.

### [Example 3]

### Lymphocyte activation experiment using spleen-derived immune cells:

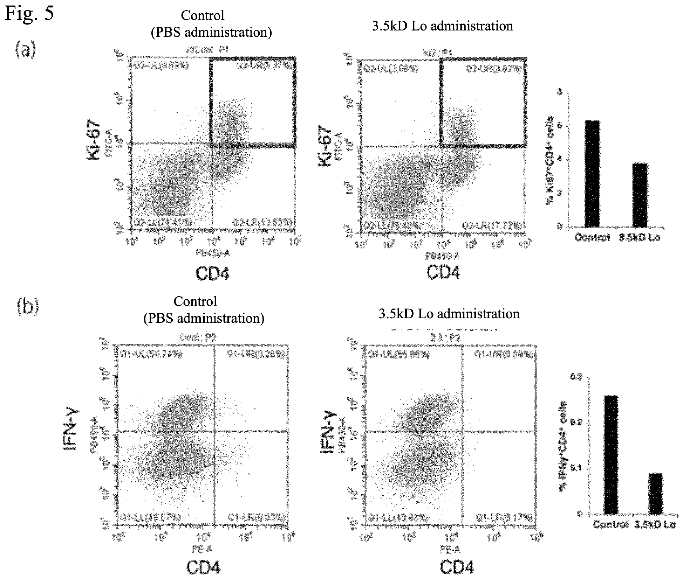

6 × 10⁶ spleen-derived immune cells were collected, and lymphocytes were activated using an anti-CD3 antibody and an anti-CD28 antibody. PBS and 3.5 kD Lo solution were each added to the medium, and the cells were then incubated for 3 days. The results obtained by examining the effects on lymphocyte proliferation and differentiation into effector helper T cells are shown in Figs. 5(a) and (b), respectively.

As a result of analysis of Ki67-positive CD4-positive T cells, which are cell proliferation markers, by flow cytometry, the Ki67-positive CD4-positive cells in the group in which 3.5 kD Lo solution was added to one-half of the total volume of the medium were 3.83%.

On the other hand, the Ki67-positive CD4-positive cells in the control group (i.e., group in which PBS was added to one-half of the medium volume) were 6.37%, and proliferation of CD4-positive T cells was suppressed in the 3.5 kD Lo group (Fig. 5(a)).

The IFNγ-positive CD4-positive Th1 cells in the group in which 3.5 kD Lo solution was added to one-half of the total volume of the medium were 0.09%, whereas the IFNγ-positive CD4-positive Th1 cells in the control group (i.e., group in which PBS was added to one-half of the medium volume) were 0.26%, and differentiation of lymphocytes into Th1 cells was suppressed in the 3.5 kD Lo group (Fig. 5(b)).

### [Industrial Applicability]

The substanceof the present invention exhibits corneal epithelial damage suppressive effect, tear volume retention effect, suppressive effect on inflammatory cell infiltration into the cornea, and corneal epithelial wound healing promoting effect and is useful for ocular GVHD and dry eye as a novel therapeutic agent and therefore has industrial applicability.

## Claims

1. An agent for treating or preventing dry eye, an agent for treating or preventing graft-versus-host disease, an agent for promoting healing of corneal epithelial wound, an agent for suppressing an inflammatory cell infiltrating into the cornea, a tear volume retaining agent, an agent for suppressing a corneal epithelial damage, an agent for suppressing a cell positive for a cell proliferation marker, or an agent for suppressing differentiation of a lymphocyte into an effector T cell, the agent comprising a substance derived from a culture supernatant of a stem cell as an active ingredient.

2. The agent of Claim 1, wherein the graft-versus-host disease is graft-versus-host disease in the eye.

3. The agent of Claim 1, wherein the stem cell is a dental pulp stem cell.

4. The agent of Claim 3, wherein the dental pulp stem cell is a deciduous tooth-derived dental pulp stem cell.

5. The agent of Claim 1, wherein the stem cell is a primary cell.

6. The agent of Claim 1, wherein the stem cell is an immortalized cell.

7. The agent of Claim 1, wherein the substance derived from the culture supernatant of the stem cell is a substance obtained by a method comprising the step of removing components of molecular weight less than 3.5 kDa from the culture supernatant of the stem cell.

8. The agent of Claim 7, wherein the method comprising the step of removing components of molecular weight less than 3.5 kDa from the culture supernatant of the stem cell is a method of using a dialysis membrane.

9. The agent of Claim 1, wherein the substance derived from the culture supernatant of the stem cell does not comprise components of molecular weight less than 3.5 kDa.

10. The agent of Claim 1, wherein the protein concentration of the substance derived from the culture supernatant of the stem cell is 30 µg/mL to 100 µg/mL.

11. The agent of Claim 1, wherein the substance derived from the culture supernatant of the stem cell comprises an extracellular vesicle.

12. The agent of Claim 1, wherein the substance derived from the culture supernatant of the stem cell comprises an exosome.
